(19) Europäisches Patentamt

European Patent Office

Office européen des brevets

(11) Veröffentlichungsnummer: **0 217 250 B1**

(12) **EUROPÄISCHE PATENTSCHRIFT**

(45) Veröffentlichungstag der Patentschrift: **08.04.92**

(21) Anmeldenummer: **86113005.2**

(22) Anmeldetag: **20.09.86**

(51) Int. Cl.⁵: **A61K 7/50**, A61K 7/08

(54) **Schäumende Tensidzubereitungen mit klar solubilisierten wasserunlöslichen Ölkomponenten.**

(30) Priorität: **28.09.85 DE 3534733**

(43) Veröffentlichungstag der Anmeldung:
**08.04.87 Patentblatt 87/15**

(45) Bekanntmachung des Hinweises auf die
Patenterteilung:
**08.04.92 Patentblatt 92/15**

(84) Benannte Vertragsstaaten:
**DE**

(56) Entgegenhaltungen:
EP-A- 0 060 372          EP-A- 0 086 878
EP-A- 0 155 737          GB-A- 1 584 127
US-A- 3 666 857          US-A- 4 329 334

HOUSEHOLD & PERSONAL PRODUCTS INDU-
STRY, Band 20, Nr. 9, September 1983, Ramsey, NJ (US); Seite 18

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Scholz, Wolfhard
Edmundstr. 26
W-4150 Krefeld 11(DE)**
Erfinder: **Schosser, Gryta
Kaiserstr. 90
W-4150 Krefeld(DE)**

**Beschreibung**

Gegenstand der Erfindung sind gut schäumende Tensidzubereitungen mit klar solubilisierten, wasserunlöslichen kosmetischen Ölkomponenten.

Tensidzubereitungen auf Basis synthetischer, stark schäumender anionischer Tenside werden in großem Umfange als Körperwasch- und Reinigungsmittel, z. B. als Shampoos, Schaumbadzubereitungen, Duschbäder, flüssige Seifen und Waschlotionen verwendet. Leider haben viele anionische Tenside die Eigenschaft, die Haut stark zu entfetten, was sich nach dem Waschen und Abtrocknen durch ein Spannen der Haut und durch ein rauhes, rissiges oder schuppiges Aussehen der Haut bemerkbar macht.

Man hat daher versucht, kosmetische Ölkomponenten als Rückfettungsmittel in solche Tensidzubereitungen einzuarbeiten. Üblicherweise geschieht die Einarbeitung durch Emulgierung unter Bildung von Öl-in-Wasser-Emulsionen. Leider wirken sich solche emulgierten Ölkomponenten sehr nachteilig auf die Schäumkraft der Zubereitungen aus. Eine weitere Möglichkeit besteht darin, Ölkomponenten unter Verwendung von organischen wasserlöslichen Lösungsmitteln, z. B. von niederen Alkoholen oder Alkylglycolen mit $C_1$-$C_4$-Alkylgruppen zu solubilisieren. Leider wirken auch solche organischen Lösungsvermittler stark schaumdämpfend.

Es bestand daher die Aufgabe, wasserunlösliche Ölkomponenten ohne solche schaumdämpfenden Lösungsvermittler klar in Tensidzubereitungen zu solubilisieren. Es wurde gefunden, daß diese Aufgabe durch eine Kombination von besonders hautverträglichen nichtionischen polyoxethylierten Emulgatoren und anionischen Tensiden gelöst werden kann.

Gegenstand der Erfindung sind wäßrige, gut schäumende Tensidzubereitungen mit klar solubilisierten, wasserunlöslichen schwerflüchtigen Ölen, dadurch gekennzeichnet, daß sie

(A) 0,05 - 3 Gew.-% eines klar solubilisierten Öls, Fettes oder Wachses

(B) 0,5 - 30 Gew.-% eines nichtionischen polyoxethylierten Emulgators, der ausgewählt ist aus der Gruppe

(B1) fettsäure-$C_8$-$C_{18}$-mono- und diester Von Anlagerungsprodukten von 5 bis 30 Mol Ethylenoxid an Glycerin

(B2) Anlagerungsprodukte von 5 bis 30 Mol Ethylenoxid an Fettsäure-$C_8$-$C_{18}$-mono-, di- und triglyceride

(B3) Anlagerungsprodukte von 1 bis 10 Mol Ethylenoxid an Fettsäure-$C_8$-$C_{18}$-mono- und diethanolamide

(B4) Anlagerungsprodukte von 2 bis 20 Mol Ethylenoxid an Oleylalkohol und/der an $C_{16}$-$C_{18}$-Fettalkoholfraktionen mit mehr als 40 Gew.-% Oleylalkohol,

wobei entweder ein Emulgator des Typs B1 oder B2 alleine oder ein Gemisch wenigstens zweier Emulgatoren verschiedenen Typs enthalten ist,

(C) 10 - 30 Gew.-% eines anionischen Sulfat- oder Sulfonattensids

(D) 0 - 10 Gew.-% eines amphoteren und/oder zwitterionischen Tensids und/oder eines Aminoxid-Tensids

(E) 40 - 80 Gew.-% Wasser

enthalten.

Unter dem klar solubilisierten Öl wird im Sinne der vorliegenden Erfindung nicht ein ätherisches Öl oder ein leichtflüchtiger Bestandteil eines ätherischen Öls oder einer natürlichen oder synthetischen Duftstoffkomposition verstanden, für die in der Literatur zahlreiche Solubilisatoren vorgeschlagen werden.

Bei der vorliegenden Erfindung geht es vielmehr darum, schwerflüchtige Ölkomponenten, Fette oder Wachse, die zur Fettung und Pflege der Haut geeignet sind, und in der Regel mehr als 12 C-Atome im Molekül aufweisen, klar zu solubilisieren. Es hat sich gezeigt, daß kosmetische Öle, die bei +20 °C noch flüssig sind, für die Herstellung der erfindungsgemäßen Zubereitungen besonders gut geeignet sind. In erfindungsgemäßen Tensidzubereitungen, in welchen das solubilisierte Öl etwa 14 bis 24 C-Atome im Molekül aufweist und bei +20 °C flüssig ist, beträgt das Gewichtsverhältnis der Komponenten A : B bevorzugt 1 : 0,5 bis 1 : 8. Solche leichter zu solubilisierende Öle sind z. B. Isopropylmyristat und Isopropylpalmitat. Andere relativ leicht zu solubilisierende Ölkomponenten sind Alkohole mit 16 bis 24 C-Atomen, z. B. 2-Hexyl-decanol, Oleylalkohol, 2-Octyldodecanol und Kohlenwasserstoffe, insbesondere verzweigte Kohlenwasserstoffe wie z. B. Isoparaffine mit 16 bis 30 C-Atomen, z. B. Paraffinöle, 1,3-Dialkylcyclohexane mit 4 bis 20 C-Atomen in der Alkylkette.

Einen höheren Anteil an nichtionischem, polyoxethyliertem Emulgator benötigen die höhermolekularen Ölkomponenten, Fette und Wachse. Unter diesen Produkten sind die bei einer Temperatur von +20 °C noch f lüssigen Öle für die Ausführung der Erfindung bevorzugt. Beispiele für solche bevorzugte kosmetische Öle sind die Fettsäure-Fettalkoholester mit 24 bis 44 C-Atomen wie z. B. Oleyloleat, Isooctylstearat, Decyloleat, Decylerucat oder natürliche Öle, die ganz oder überwiegend aus solchen flüssigen Wachsestern bestehen, z. B. Spermöl und Jojobaöl und synthetische Ester, deren Zusammensetzung diesen natürlichen Ölen weitgehend entspricht. Weitere Beispiele sind Fettsäuretriglyceride auf Basis von $C_8$-$C_{22}$-Fettsäuren, z. B. Capryl-und/oder Caprinsäuretriglycerid, Ölsäuretriglycerid, flüssige natürliche Triglyceridöle wie z. B. Olivenöl, Sonnenblumenöl, Mandelöl, Avocadoöl, Dicarbonsäureester wie z. B. Dibutylsebacat, Dioctyladipat, die Vollester von Diolen und Polyolen wie z. B. die Fettsäure-$C_{12}$-$C_{22}$-ester des Ethylenglycols, der Propylenglycole, des Hexandiols, des Butandiols, des Neopentylglycols, des Trimethylolpropans, des Pentaerythrits, die Komplexester aus Dicarbonsäuren, Polyolen und Fettsäuren sowie Weizenkeimöl, Vitamin E (Tocopherol) und Tocopherolacetat. In erfindungsgemäßen Tensidzubereitungen, in welchen das solubilisierte Öl mehr als 24 C-Atome aufweist und bei +20 °C flüssig ist, beträgt das Gewichtsverhältnis der Komponenten A : B bevorzugt 1 : 5 bis 1 : 16.

Wenn die zu solubilisierende Komponente (A) ein bei 20 °C nicht mehr flüssiges Produkt, also z. B. eine Fettkomponente oder ein natürliches oder synthetisches Wachs darstellt, so wird dieses bevorzugt in kleineren Mengen, etwa im Bereich von 0,05 bis 1 Gew.-% der Zubereitung eingesetzt. Geeignete kosmetische Fette und Wachse sind z. B. Wollfett, Bürzeldrüsenfett, Vaseline, gehärtete Pflanzenfette, Bienenwachs, Candelillawachs, Carnaubawachs, Montanwachs, Hartparaffin, Japanwachs und synthetische Ester, deren Zusammensetzung und Eigenschaften diesen natürlichen Fetten und Wachsen entspricht. Fette und Wachse, die für die Ausführung der Erfindung geeignet sind, sollten einen Schmelzpunkt unterhalb 75 °C aufweisen.

Der nichtionische polyethoxylierte Emulgator ist gekennzeichnet durch wenigstens eine lineare Alkyl- oder Acylgruppe mit 8 bis 22 C-Atomen und durch eine hydrophile Gruppe, die wenigstens eine Polyoxyethylgruppe aus wenigstens vier Oxyethyleinheiten aufweist. Als nichtionische polyoxethylierte Emulgatoren eignen sich solche aus der Gruppe

(B1) Fettsäure-$C_8$-$C_{18}$-mono- und diester von Anlagerungsprodukten von 4 bis 20 Mol Ethylenoxid an Glycerin, wie sie z. B. aus DE-A 20 24 051 bekannt sind.

(B2) Anlagerungsprodukte von 4 bis 20 Mol Ethylenoxide an Fettsäure-$C_8$-$C_{18}$-mono-, di- und triglyceride. Solche Anlagerungsprodukte an Partialglyceridgemischen von gesättigten Pflanzenfettsäuren sind z. B. aus DE-A 14 67 816 bekannt. Ethylenoxid-Anlagerungsprodukte an hydriertes Ricinusöl, also an ein Hydroxystearinsäure-triglycerid, sind aus DE-A 15 92 996 bekannt.

(B3) Anlagerungsprodukte von 1 bis 10 Mol Ethylenoxid an Fettsäure-$C_8$-$C_{18}$-mono- und diethanolamide. Solche Produkte sind z. B. aus DE-A 18 06 010 bekannt.

(B4) Anlagerungsprodukte von 2 bis 10 Mol Ethylenoxid an Oleylalkohol und/oder $C_{16}$-$C_{18}$-Fettalkoholfraktionen mit mehr als 40 Gew.-% Oleylalkohol.

Die nichtionischen polyoxethylierten Emulgatoren des Typs B1 und B2, deren grenzflächenaktive Eigenschaften relativ schwach ausgeprägt sind und die selbst gewisse rückfettende Eigenschaften aufweisen, eignen sich am besten für die Herstellung der erfindungsgemäßen Tensidzubereitungen. Sie können zur Herstellung der erfindungsgemäßen Tensidzubereitungen allein, d. h. ohne weitere nichtionische Emulgatoren verwendet werden. Wird ein Emulgator des Typs B1 oder B2 nicht eingesetzt, so ist es erforderlich, ein Gemisch wenigstens zweier Emulgatoren verschiedenen Typs einzusetzen. Es ist selbstverständlich auch möglich, nichtionische Emulgatoren des Typs B1 oder B2 gemeinsam mit solchen des Typs B3 und/oder B4 oder mit an deren nichtionischen Emulgatoren einzusetzen.

Solche anderen nichtionischen Emulgatoren sind z. B. die Anlagerungsprodukte von 4 bis 20 Mol Ethylenoxid an Alkylphenol mit 8 bis 15 C-Atomen in der Alkylgruppe, an Fettamine mit 8 bis 22 C-Atomen, an Fettsäuren mit 12 bis 22 C-Atomen oder an Sorbitan-Fettsäure-$C_{12-22}$-Partialester.

Die anionischen Tenside sind gekennzeichnet durch eine lineare Alkylgruppe mit 8 bis 22 C-Atomen und eine $SO_3^{(-)}$ oder -O-$SO_3^{(-)}$-Gruppe im Molekül und liegen in Form eines wasserlöslichen Salzes, z. B. eines Alkali-, Magnesium-, Ammonium-, Mono-, Di- oder Trialkanolammoniumsalzes mit 2 bis 4 C-Atomen in der Alkanolgruppe vor.

Bevorzugt als stark schäumende anionische Tenside sind die Lithium-, Natrium-, Kalium-, Ammonium-, Magnesium-, Mono-, Di-oder Triethanolammoniumsalze von

(C1) Alkylsulfaten und/oder Alkylpolyglycolethersulfaten der allgemeinen Formel $R^1$-O-$(CH_2$-$CH_2O)_x$-$SO_3^{(-)}$, in der $R^1$ eine lineare Alkylgruppe mit 10 bis 16 C-Atomen, x = 0 oder eine Zahl von 0 bis 20 ist

(C2) linearen Alpha-Olefinsulfonaten mit 12 bis 18 C-Atomen

(C3) linearen sekundären Alkansulfonaten mit 12 bis 18 C-Atomen.

Weitere geeignete stark schäumende anionische Sulfat- oder Sulfonattenside sind die Salze von Sulfobernsteinsäuremono-und dialkylestern mit 6 bis 18 C-Atomen in den Alkylgruppen, von Sulfobernsteinsäuremono(polyethoxyalkyl)estern mit 1 bis 6 Ethoxygruppen und 10 bis 16 C-Atomen in den Alkylgruppen, von Fettsäurealkanolamidpolyglycolethersulfaten der allgemeinen Formel $R^2CONH(CH_2$-

$CH_2O)_x$-$SO_3^{(-)}$, in der $R^2$-CO eine Acylgruppe mit 8 bis 18 C-Atomen und x = 1 bis 6 ist, von Fettsäure-$C_{12}$-$C_8$-alpha-sulfonaten, von Acyl ($C_8$-$C_{18}$)-isethionaten und von Acyl-($C_8$-$C_{18}$)-tauriden.

Obwohl für die Solubilisierung des wasserunlöslichen Öles nicht erforderlich, enthalten die erfindungsgemäßen Tensidzubereitungen bevorzugt einen Zusatz von amphoteren und/oder zwitterionischen Tensiden und/oder eines Aminoxid-Tensids (Komponente D) zur weiteren Verbesserung der Haut- und Schleimhautverträglichkeit. Das Gewichtsverhältnis der Komponenten C : D ist bevorzugt etwa 1 : 0,1 bis 1 : 0,5.

Als amphotere Tenside werden solche verstanden, die neben einer Alkyl- oder Acylgruppe mit 12 bis 18 C-Atomen eine Aminogruppe und eine Carbonsäure- oder Sufonsäuregruppe im Molekül tragen. Beispiele für solche amphotere Tenside sind z. B. N-($C_{12}$-$C_{18}$-alkyl)-$\beta$-aminopropionsäure, N-($C_8$-$C_{18}$-alkyl)-$\beta$-aminobuttersäure, N-($C_8$-$C_{18}$-alkyl)-iminodiessigsäure, N-($C_{12}$-$C_{16}$-alkyl)-N-2-hydroxyethyl-glycin, N-($C_{12}$-$C_{18}$-alkyl)-taurin, N-($C_8$-$C_{18}$)-alkyl benzylamin-p-sulfonsäure, N-($C_8$-$C_{18}$-alkyl)-1-aminobutan-3-sulfonsäure.

Als zwitterionische Tenside sind solche zu verstehen, die neben einer Alkyl- oder Acylgruppe mit 12 bis 18 C-Atomen eine quartäre Ammoniumgruppe und eine Carboxylat- oder Sulfonatgruppe im Molekül enthalten. Beispiele für solche zwitterionischen Tenside sind z. B. N-($C_8$-$C_{18}$-alkyl)-N,N-dimethyl-ammonioglycinat, N-Kokosacylamidopropyl-N,N-dimethyl-ammonioglycinat, N-($C_8$-$C_{18}$alkyl)-N-2-hydroxyethyl-N-methyl-$\beta$-ammoniopropionat, N-($C_8$-$C_{18}$-alkyl)-N,N-dimethyl-$\beta$-ammoniopropansulfonat.

Aminoxid-Tenside tragen neben einer Alkyl- oder Acylgruppe mit 12 bis 18 C-Atomen eine tertiäre Aminoxid-Gruppe, z. B. N-($C_8$-$C_{18}$-alkyl)-N,N-dimethylaminoxid oder N-$C_8$-$C_{18}$-acylaminopropyl-N,N-dimethyl-aminoxid.

Die Herstellung der erfindungsgemäßen Tensidzubereitungen ist von erheblicher Bedeutung für die klare Solubilisierung der Öle, Fette oder Wachse. Es wurde gefunden, daß eine klare Solubilisierung nur gelingt, wenn man das Öl, Fett oder Wachs (A) und den nichtionischen polyoxethylierten Emulgator (B) mischt und gegebenenfalls die Mischung über ihren Schmelzpunkt bis zur Bildung einer homogenen Vormischung erwärmt, in die Vormischung eine auf die gleiche Temperatur erwärmte wäßrige Lösung des anionischen Tensids (C) und gegebenenfalls des amphoteren und/oder zwitterionischen Tensids und/oder des Aminoxid-Tensids (D) einmischt und gegebenenfalls, nach dem Abkühlen, weiteres Wasser und weitere übliche kosmetische Zusatzstoffe einmischt.

Wenn man hingegen die kosmetischen Öle, Fette oder Wachse nicht mit dem nichtionischen, polyoxethylierten Emulgator vormischt, sondern separat der wäßrigen Tensidzubereitung zusetzt, wird eine klare Solubilisierung nicht erreicht. Unter den gegebenenfalls in erfindungsgemäßen Tensidzubereitungen enthaltenen üblichen kosmetischen Zusatzstoffen werden in erster Linie Hilfsmittel zur Verbesserung der Lagerstabilität, zur Verbesserung von Aussehen und Geruch, zur Erhöhung der Viskosität und zur Verbesserung der kosmetischen Wirkung verstanden.

Beispiele für solche Zusatzstoffe, die in untergeordneten Mengen bis zu insgesamt etwa 10 Gew.-% in den erfindungsgemäßen Tensidzubereitungen enthalten sein können, sind Konservierungsmittel wie z. B. Formaldehyd, p-Hydroxybenzoesäuremethylester, Sorbinsäure, Natriumbenzoat, 5-Brom-5-Nitro-1,3-dioxan, 5-Chlor-2-methyl-4-isothiazolin-3-on und/oder 2-Methyl-4-isothiazolin-3-on und Verdickungsmittel, z. B. wasserlösliche Polymere wie Carboxymethylcellulose, Hydroxyethylcellulose, vernetzte Carboxyvinylpolymerisate (z. B. die Handelsprodukte Carbopol ® ) Fettsäure $C_{12}$-$C_{18}$-mono- und diethanolamid und die mit solchen Fettsäurealkanolamiden synergistisch wirksamen Salze wie z. B. Kochsalz oder Natriumsulfat, Farbstoffe und Duftstoffe, Trübungs- und Perlglanzmittel, Puffersubstanzen zur Einstellung des pH-Wertes auf Werte zwischen 5 und 8, z. B. Citronensäure und/oder Natriumcitrat, hautkosmetische Wirkstoffe wie z. B. wasserlösliche Proteine, Kräuterextrakte, kationische Polymere, Vitamine, Sebostatika, Hautfeuchthaltemittel und haarkonditionierende Zusätze.

Von besonderem Vorteil sind die erfindungsgemäßen Tensidzubereitungen, wenn diese durch Perlglanzmittel oder Trübungsmittel ein ästhetisch ansprechendes Aussehen erhalten sollen, da emulgierte bzw. dispergierte Öle, Fette und Wachse den Perlglanz und die Stabilität solcher Zubereitungen erheblich beeinträchtigen.

Erfindungsgemäße Tensidzubereitungen können durch einen Zusatz von 0,1 bis 2 Gew.-% eines Esters der allgemeinen Formel $R^3$-(OC$_n$H$_{2n}$)$_x$OR$^4$, in der $R^3$ eine lineare Fettacylgruppe mit 16 bis 22 C-Atomen und $R^4$ Wasserstoff oder eine Fettacylgruppe $R^3$, n = 2 oder 3 und x eine Zahl von 1 bis 4 ist oder eines Gemisches eines solchen Esters mit einem Monoethanolamid von Fettsäuren mit 12 bis 22 C-Atomen ein brillant perlglänzendes Aussehen erhalten. Geeignete Ester sind z. B. Ethylenglycoldistearat und Triethylenglycoldistearat.

Als Trübungsmittel werden üblicherweise Polymerdispersionen, z. B. Polystyrol-copolymerisat-Dispersionen (Opacifier ® -Typen, Antara ® -Typen) zugesetzt.

Die folgenden Beispiele sollen den Gegenstand der Erfindung näher erläutern:

4

B e i s p i e l e

| Schäumender Badezusatz | 1 | 2 | 3 | 4 | 5 | 6 | 7 | 8 | 9 |
|---|---|---|---|---|---|---|---|---|---|
| **(A)** | | | | | | | | | |
| Jojobaöl | 0,3 | 0,3 | 0,6 | 1,8 | 2,4 | - | - | - | - |
| Mandelöl | - | - | - | - | - | 0,2 | - | - | - |
| Avocadoöl | - | - | - | - | - | - | 0,2 | - | - |
| Paraffinöl (dünnflüssig) | - | - | - | - | - | - | - | 0,3 | - |
| Isopropylmyristat | - | - | - | - | - | - | - | - | 3 |
| **(B)** | | | | | | | | | |
| Cetiol® HE [1] | 3 | - | 2 | 18 | 12 | 1 | 1 | 1 | 1 |
| Eumulgin® C4 [2] | - | 2 | 2 | - | 12 | 1 | 1 | 1 | 1 |
| Eumulgin® O5 [3] | - | 2 | 2 | - | - | 1 | 1 | 1 | 1 |
| **(C)** | | | | | | | | | |
| Sulfopon® 101 [5] | - | - | - | - | - | - | - | - | 60 |
| Texapon® N25 [4] | 55 | 55 | 77 | 55 | 55 | 55 | 60 | 60 | - |
| Hostapur® OS fl. [6] | - | - | - | - | - | 5 | - | - | - |
| Elfan® OS46 [7] | 5 | 5 | - | 5 | 5 | - | - | - | - |
| **(D)** | | | | | | | | | |
| Dehyton® K [8] | 5 | 5 | - | 5 | 5 | 5 | 5 | 5 | 5 |
| Aminoxid WS35 [9] | 1 | 1 | - | - | - | - | - | - | - |
| Polymer JR 400 [11] | - | - | - | - | - | 0,3 | - | - | 0,3 |
| Merquat® 550 [10] | 0,5 | 0,5 | - | - | - | - | 0,5 | 0,5 | - |
| Duftstoff | 1,2 | 1,2 | 1,2 | 1,2 | 1,2 | 1,2 | 1,2 | 1,2 | 1,2 |
| Wasser | ad 100 | ad 100 | ad 100 | ad 100 | ad 100 | ad 100 | ad 100 | ad 100 | ad 100 |

Herstellung:

Die Rezepturen für schäumende Badezusätze 1 bis 9 wurden wie folgt hergestellt:
Die Ölkomponente (A) und ggf. die polyoxethylierten Emulgatoren (B) wurden gemischt und ggf. auf 40 °C erwärmt. In die klare Mischung wurden die wäßrigen Lösungen der anionischen Tenside (C) und ggf. des zwitterionischen Tensids und des Aminoxids eingerührt und nach dem Abkühlen auf 20 °C wurde ggf. kationisches Polymer sowie der Duftstoff und das restliche Wasser unter Rühren zugesetzt. Es wurden klare und stabile gut schäumende Zubereitungen erhalten.

Vergleichsversuche:

EP 0 217 250 B1

Bei allen Rezepturen wurde versucht, die Ölkomponente (A) ohne Vormischung von (A) und (B) einzuarbeiten. In diesen Fällen wurden trübe Zubereitungen erhalten.

In den Rezepturen 1 bis 9 wurden die folgenden Warenzeichen verwendet:

1) Cetiol ® HE: Veresterungsprodukt aus 1 Mol Kokosfettsäure-$C_8$-$C_{18}$ und 1 Mol eines Anlagerungsproduktes von 7,4 Mol Ethylenoxid an Glycerin

2) Eumulgin ® C4: Anlagerungsprodukt von 4 Mol Ethylenoxid an Kokosfettsäure-$C_{12}$-$C_{18}$-monoethanolamid

3) Eumulgin ® O5: Anlagerungsprodukt von 5 Mol Ethylenoxid an ein Oleyl-Cetylalkohol-Gemisch

4) Texapon ® N25: 28%ige wäßrige Lösung eines Fettalkohol-$C_{12}$-$C_{14}$-polyglycolether(2EO)-sulfat-Na-Salzes

5) Sulfopon ® 101: 28%ige wäßrige Lösung eines Fettalkohol-$C_{12}$-$C_{16}$-sulfat-Na-Salzes

6) Hostapur ® OS fl.: 40%ige wäßrige Lösung eines linearen $C_{15}$-$C_{18}$-$\alpha$-Olefinsulfonat, Na-Salzes (Hoechst)

7) Elfan ® OS46: 36%ige wäßrige Lösung eines linearen $C_{14}$-$C_{16}$-$\alpha$-Olefinsulfonat, Na-Salzes (Akzo)

8) Dehyton ® K: 30%ige wäßrige Lösung eines N-Kokosacylamidopropyl-N,N-dimethyl-ammonioglycinats (Henkel KGaA)

9) Aminoxid WS35: 35%ige wäßrige Lösung eines N-Kokosacylamidopropyl-N,N-dimethyl-aminoxids (Goldschmidt)

10) Merquat ® 550: 8%ige wäßrige Lösung eines Dimethyldiallylammoniumchlorid-Acrylamid-Copolymers (Merck Chem. Div.)

11) Polymer JR ® 400: Kationisches Cellulosederivat (CTFA-Bezeichnung: Polyquaternium-10)

**Patentansprüche**

1. Wäßrige, gut schäumende Tensidzubereitungen, gekennzeichnet durch einen Gehalt von
   (A) 0,05 bis 3 Gew.-% eines klar solubilisierten, wasserunlöslichen Öls, Fettes oder Wachses
   (B) 0,5 bis 30 Gew.-% eines nichtionischen, polyoxethylierten Emulgators B, der ausgewählt ist aus der Gruppe
       (B1) Fettsäure-$C_8$-$C_{18}$-mono- und diester von Anlagerungsprodukten von 5 bis 30 Mol Ethylenoxid an Glycerin
       (B2) Anlagerungsprodukte von 5 bis 30 Mol Ethylenoxid an Fettsäure-$C_8$-$C_{18}$-mono-, di- und triglyceride
       (B3) Anlagerungsprodukte von 1 bis 10 Mol Ethylenoxid an Fettsäure-$C_8$-$C_{18}$-mono- und diethanolamide
       (B4) Anlagerungsprodukte von 2 bis 20 Mol Ethylenoxid an Oleylalkohol und/ oder an $C_{16}$-$C_{18}$-Fettalkoholfraktionen mit mehr als 40 Gew.-% Oleylalkohol,
   wobei entweder ein Emulgator des Typs B1 oder B2 alleine oder ein Gemisch wenigstens zweier Emulgatoren verschiedenen Typs enthalten ist,
   (C) 10 bis 30 Gew.-% eines anionischen Sulfat- oder Sulfonattensids
   (D) 0 bis 10 Gew.-% eines amphoteren und/oder zwitterionischen Tensids und/oder eines Aminoxid-Tensids
   (E) 40 bis 80 Gew.-% Wasser.

2. Tensidzubereitungen nach Anspruch 1, dadurch gekennzeichnet, daß die Komponente (A) ein bei +20°C flüssiges kosmetisches Öl ist.

3. Tensidzubereitungen nach den Ansprüchen 1 oder 2, dadurch gekennzeichnet, daß das anionische Tensid (C) ausgewählt ist aus der Gruppe der Lithium-, Natrium-, Kalium-, Ammonium-, Magnesium-, Mono-, Di- oder Triethanolammoniumsalze von
   (C1) Alkylsulfaten und Alkylpolyglycolethersulfaten der allgemeinen Formel $R^1$-O(CH2-$CH_2$-O)$_x$$SO_3$(-), in der $R^1$ eine lineare Alkylgruppe mit 10 bis 16 C-Atomen, x = 0 oder eine Zahl von 1 bis 20 ist
   (C2) linearen alpha-Olefinsulfonaten mit 12 bis 18 C-Atomen oder
   (C3) linearen sekundären Alkansulfonaten mit 12 bis 18 C-Atomen.

4. Tensidzubereitungen nach den Ansprüchen 1 bis 3, dadurch gekennzeichnet, daß das solubilisierte Öl bei +20°C flüssig ist und mehr als 24 C-Atome enthält und das Gewichtsverhältnis der Komponenten A : B = 1 : 5 bis 1 : 16 beträgt.

6

**5.** Tensidzubereitungen nach den Ansprüchen 1 bis 4, dadurch gekennzeichnet, daß das Gewichtsverhältnis der Komponenten C : D = 1 : 0,1 bis 1 : 0,5 beträgt.

**6.** Verfahren zur Herstellung von Tensidzubereitungen nach einem der Ansprüche 1 bis 5, dadurch gekennzeichnet, daß man das Öl, Fett oder Wachs (A) und den nichtionischen polyoxethylierten Emulgator (B) mischt und die Mischung gegebenenfalls über ihren Schmelzpunkt bis zur Bildung einer homogenen Vormischung erwärmt, in die Vormischung eine auf die gleiche Temperatur erwärmte, wäßrige Lösung des anionischen Tensids (C) und gegebenenfalls des amphoteren und/oder zwitterionischen Tensids und/oder des Aminoxid-Tensids (D) einmischt und gegebenenfalls, nach dem Abkühlen, weiteres Wasser und weitere übliche Zusatzstoffe einmischt.

## Claims

**1.** Aqueous, high-foaming surfactant preparations characterized by a content of
(A) 0.05 to 31% by weight of a clearly solubilized water-insoluble oil, fat or wax,
(B) 0.5 to 30% by weight of a nonionic polyethoxylated emulsifier B selected from the group consisting of
(B1) fatty acid-$C_{8-18}$-monoesters and diesters of adducts of 5 to 30 mol ethylene oxide with glycerol,
(B2) adducts of 5 to 30 mol ethylene oxide with fatty acid-$C_{8-18}$-mono-, di- and triglycerides,
(B3) adducts of 1 to 10 mol ethylene oxide with fatty acid-$C_{8-18}$-mono- and diethanolamides and
(B4) adducts of 2 to 20 mol ethylene oxide with oleyl alcohol and/or with $C_{6-18}$ fatty alcohol fractions containing more than 40% by weight oleyl alcohol,
either an emulsifier of the type B1 or B2 alone or a mixture of at least two emulsifiers of different types being present,
(C) 10 to 30% by weight of an anionic sulfate or sulfonate surfactant,
(D) 0 to 10% by weight of an amphoteric and/or zwitter-ionic surfactant and/or an amine oxide surfactant
(E) 40 to 80% by weight water.

**2.** Surfactant preparations as claimed in claim 1, characterized in that component (A) is a cosmetic oil liquid at $+20\,^\circ$C.

**3.** Surfactant preparations as claimed in claim 1 or 2, characterized in that the anionic surfactant (C) is selected from the group consisting of the lithium, sodium, potassium, ammonium, mangesium, mono-, di- or triethanolammonium salts of
(C1) alkyl sulfates or alkyl polyglycol ether sulfates corresponding to the general formula

$$R^1O(CH_2\text{-}CH_2\text{-}O)_x\text{-}SO_3{}^{(-)},$$

in which R' is a linear alkyl group containing 10 to 16 carbon atoms, x = 0 or a number of 1 to 20,
(C2) linear $\alpha$-olefinsulfonates containing 12 to 18 carbon atoms or
(C3) linear secondary alkanesulfonates containing 12 to 18 carbon atoms.

**4.** Surfactant preparations as claimed in claims 1 to 3, characterized in that the solubilized oil is liquid at $+20\,^\circ$C and contains more than 24 carbon atoms and the ratio by weight of component A to component B is from 1:5 to 1:16.

**5.** Surfactant preparations as claimed in claims 1 to 4, characterized in that the ratio by weight of component C to component D is from 1:0.1 to 1:0.5.

**6.** A process for the production of the surfactant preparations claimed in any of claims 1 to 5, characterized in that the oil, fat or wax (A) and the nonionic polyethoxylated emulsifier (B) are mixed and the resulting mixture is optionally heated to beyond its melting point until a homogeneous premix is formed, an aqueous solution - heated to the same temperature - of the anionic surfactant (C) and, optionally, the amphoteric and/or zwitter-ionic surfactant and/or the amine oxide surfactant (D) is introduced into the premix and, after cooling, more water and other standard additives are optionally added.

**Revendications**

1.  Préparations aqueuses bien moussantes d'agents tensio-actifs caractérisées par une teneur de

    (A) 0,05 à 3 % en poids d'une huile, graisse ou cire solubilisée clairement, insoluble dans l'eau,

    (B) 0,5 à 30 % en poids d'un émulsionnant B polyoxyéthylé non ionique qui est choisi dans le groupe

    (B1) des monoesters et diesters d'acide gras $C_8$-$C_{18}$ de produits d'addition de 5 à 30 moles d'éthylèneoxyde à du glycérol;

    (B2) des produits d'addition de 5 à 30 modes d'éthylèneoxyde à des monoglycérides, diglycérides et triglycérides d'acide gras $C_8$-$C_{18}$

    (B3) des produits d'addition de 1 à 10 moles d'éthylèneoxyde à des monoéthanolamides et diéthanolamides d'acide gras $C_8$-$C_{18}$

    (B4) des produits d'addition de 2 à 20 moles d'éthylèneoxyde et d'alcool oléylique et/ou de fractions d'alcool gras $C_{16}$-$C_{18}$ avec plus de 40 % en poids d'alcool oléylique,

    un émulsionnant de type B1 ou B2, seul ou un mélange d'au moins deux émulsionnants de types différents étant contenus,

    (C) 10 à 30 % en poids d'un agent tensio-actif anionique de sulfate ou de sulfonate

    (D) 0 à 10 % en poids d'un agent tensio-actif amphotère et/ou zwitterionique et/ou d'un agent tensio-actif d'aminoxyde

    (E) 40 à 80 % en poids d'eau.

2.  Préparations d'agents tensio-actifs selon la revendication 1 caractérisées en ce que l'élément (A) est une huile cosmétique liquide à + 20°C.

3.  Préparations d'agents tensio-actifs selon les revendications 1 ou 2 caractérisées en ce que l'agent tensio-actif anionique (C) est choisi à partir du groupe des sels de lithium, de sodium, de potassium, d'ammonium, de magnésium, de monoéthanolammonium, de diéthanolammonium ou de triéthanolammonium

    (C1) d'alkylsulfates et d'alkylpolyglycoléthersulfates de formule générale $R^1$-$O(CH_2$-$CH_2$-$O)_x$-$SO_3$(-) dans laquelle $R^1$ désigne un radical alkyle linéaire avec 10 à 16 atomes C, x = 0 ou un nombre de 1 à 20

    (C2) d'alpha-oléfinesulfonates linéaires avec 12 à 18 atomes C
        ou
    (C3) d'alcanesulfonates secondaires linéaires avec 12 à 18 atomes C.

4.  Préparations d'agents tensio-actifs selon les revendications 1 à 3 caractérisées en ce que l'huile solubilisée est liquide à +20°C et contient plus de 24 atomes C et en ce que le rapport pondéral des éléments A :B est de l'ordre de 1 : 5 à 1 : 16.

5.  Préparations d'agents tensio-actifs selon les revendications 1 à 4 caractérisées en ce que le rapport pondéral des éléments C : D est de l'ordre de 1 : 0,1 à 1 : 0,5.

6.  Procédé de préparation d'agents tensio-actifs selon une des revendications 1 à 5 caractérisé en ce que l'huile, la graisse et la cire (A) et l'émulsionnant polyoxyéthylé non ionique (B) sont mélangés, en ce que le mélange est chauffé éventuellement jusqu'à une température supérieure à son point de fusion jusqu'à formation d'un prémélange homogène, une solution aqueuse chauffée à même température de l'agent tensio-actif anionique (c) et, éventuellement, de l'agent tensio-actif amphotère et/ou zwitterionique et/ou de l'agent tensio-actif aminoxide (D) est incorporée dans le prémélange et éventuellement, après refroidissement, de l'eau et des additifs ordinaires supplémentaires y sont incorporés.